# EUROPEAN PATENT SPECIFICATION

(11) **EP 2 594 235 B1**
(45) Date of publication and mention of the grant of the patent: **27.05.2015**
(21) Application number: 12191640.7
(22) Date of filing: 07.11.2012
(51) Int. Cl.: A61F 7/02, A61F 7/00

(54) **Liquid retainer and cooling and heating apparatus**
Flüssigkeitshalter und Kühl- und Heizvorrichtung
Dispositif de retenue de liquide et appareil de chauffage et de refroidissement

(30) Priority: 16.11.2011 JP 2011250270; 24.04.2012 JP 2012098609
(43) Date of publication of application: 22.05.2013
(73) Proprietor: Light Optical Works, Ltd., Suwa-City, Nagano 392-0015 (JP)
(72) Inventor: Iwanami, Masatomi, Suwa-shi Nagano 392-0015 (JP); Urai, Katsuji, Suwa-shi Nagano 392-0015 (JP)
(74) Representative: Klunker . Schmitt-Nilson . Hirsch

(56) References cited:
- EP-A1- 2 269 546
- DE-A1- 3 707 674
- JP-A- H11 269 747
- JP-A- 2002 240 898
- US-A1- 2007 068 651
- US-A1- 2008 269 852
- US-A1- 2010 210 982
- US-B1- 6 349 412

## Description

### FIELD OF THE INVENTION

The present invention relates to a liquid retainer capable of holding liquid therein. Further, the present invention also relates to a cooling and heating apparatus and a liquid transfer apparatus each including the liquid retainer.

### BACKGROUND OF THE INVENTION

Conventionally, there has been proposed a helmet-like head cooling device for cooling the head of a patient (for example, refer to Japanese Patent Application Laid-open No. 2001-258924). The head cooling device described in Japanese Patent Application Laid-open No. 2001-258924 includes a plurality of tubular cooling bags arranged in parallel to one another, a cooling water supply bag connected to one end of each of the tubular cooling bags, and a cooling water discharge bag connected to another end of each of the tubular cooling bags. The tubular cooling bags include an upper cloth, a lower cloth, and connecting strands for connecting the upper cloth and the lower cloth to each other. A tubular shape of the tubular cooling bags is formed by sewing together an edge of the upper cloth and an edge of the lower cloth, and the head of a patient is cooled by cooling water passing through the tubular cooling bags.

Further, conventionally, there has been known a liquid transfer apparatus for transferring liquid by utilizing a siphon principle (for example, refer to Japanese Patent Application Laid-open No. 2002-240898). In the liquid transfer apparatus described in Japanese Patent Application Laid-open No. 2002-240898, a hose leading to a supply bath and a hose leading to a receiving bath are connected to each other through intermediation of a double tube. Further, the hose leading to the supply bath and the double tube are connected to each other through intermediation of a valve, and the hose leading to the receiving bath and the double tube are connected to each other through intermediation of another valve.

In the head cooling device described in Japanese Patent Application Laid-open No. 2001-258924, the tubular shape of the tubular cooling bags which allow cooling water to pass therethrough is formed by sewing together the edge of the upper cloth and the edge of the lower cloth. Further, in this head cooling device, the tubular cooling bags are arranged in parallel to one another, and the cooling water supply bag and the cooling water discharge bag are connected respectively to both the ends of the tubular cooling bags. In this way, the structure of the head cooling device is complicated, and hence cost of the device may increase.

Meanwhile, in the liquid transfer apparatus described in Japanese Patent Application Laid-open No. 2002-240898, the hose leading to the supply bath and the hose leading to the receiving bath are connected to each other through intermediation of the double tube. Further, the hose leading to the supply bath and the double tube are connected to each other through intermediation of the valve, and the hose leading to the receiving bath and the double tube are connected to each other through intermediation of the another valve. In this way, the structure of the liquid transfer apparatus is complicated, and hence there is a disadvantage that the apparatus is large in size.

In order to avoid leakage problems in a cooling system for use by medical personnel during surgery US 6 349 412 B1 proposes a vest that includes a sealed, coolant receiving space through which coolant may pass. A source of liquid coolant at ambient pressure is connected to the space and a suction producing device is connected to an outlet for the space for drawing coolant from the source through the space at sub-atmospheric pressure. The vest may be formed of two flexible membranes sealed to each other to define the space and is provided with a coolant permeable spacer within the coolant space within the vest to prevent the membranes from collapsing upon each other.

### SUMMARY OF THE INVENTION

In view of the above-mentioned circumstances, the present invention has an object of providing a liquid retainer having a simple structure capable of cooling and warming a part of a human body, predetermined devices, and the like, and providing a cooling and heating apparatus including this liquid retainer. Further, the present invention has another object of providing a liquid retainer for use in a liquid transfer apparatus for transferring liquid by utilizing a siphon principle, which contributes to simplification and downsizing of the structure of the liquid transfer apparatus, and providing a liquid transfer apparatus including this liquid retainer. The invention is defined in appended independent claim 1. Preferred embodiments are described in the dependent claims.

In order to achieve the above-mentioned objects, according to an exemplary embodiment of the present invention, there is provided a liquid retainer, including: a pouch having water blocking properties and flexibility and formed into a pouch shape; and a three-dimensional knit including: a plurality of mesh-like knit garments arranged substantially parallel to each other; and a plurality of coupling strands for coupling the plurality of mesh-like knit garments to each other. The plurality of coupling strands are made of elastic chemical fiber. The three-dimensional knit is formed into a substantially flat-plate-like shape and arranged in the pouch. The three-dimensional knit retains liquid in the pouch. In the present invention, for example, the liquid retained by the three-dimensional knit in the pouch is one of cooling liquid and heating liquid.

According to the present invention, the substantially flat-plate-like three-dimensional knit including the plurality of mesh-like knit garments and the plurality of chemical-fiber coupling strands for coupling the plurality of mesh-like knit garments to each other is arranged in the pouch having water blocking properties and flexibility. In this way, the liquid retainer is formed. Further, liquid is retained by the substantially flat-plate-like three-dimensional knit in the pouch. Thus, according to the present invention, with the use of the liquid retained by the substantially flat-plate-like three-dimensional knit in a simple structure using the substantially flat-plate-like three-dimensional knit and the pouch, a part of a human body, predetermined devices, and the like can be cooled and warmed.

Further, according to the present invention, the plurality of mesh-like knit garments are coupled to each other by the plurality of elastic chemical-fiber coupling strands. Thus, the liquid can be retained between the plurality of mesh-like knit garments with a predetermined retention force by utilizing surface tension of the liquid. Therefore, even when the liquid retainer is used while standing the pouch, the liquid can be retained between the plurality of mesh-like knit garments of the substantially flat-plate-like three-dimensional knit so that the liquid can be prevented from pooling on a lower side of the pouch. As a result, according to the present invention, even when the liquid retainer is used while standing the pouch, a cooling effect or a heating effect can be yielded in all the parts corresponding to the substantially flat-plate-like three-dimensional knit.

Still further, according to the present invention, the three-dimensional knit formed into the substantially flat-plate-like shape is arranged in the pouch. Thus, when the liquid retainer is used as a cooling pillow for cooling the head of a sleeper, the head laid on the liquid retainer can be supported with the plurality of elastic chemical-fiber coupling strands. Therefore, a pressure increase in the pouch can be suppressed by the plurality of elastic chemical-fiber coupling strands. Yet further, according to the present invention, the head laid on the liquid retainer can be supported with the plurality of elastic chemical-fiber coupling strands, and hence comfort to the head laid on the liquid retainer can be improved.

Yet further, according to the present invention, even when the liquid retainer is used while standing the pouch, the liquid can be retained between the plurality of mesh-like knit garments of the substantially flat-plate-like three-dimensional knit. Thus, with use of a liquid retainer including a pouch opened on one end side and another end side of the liquid retainer, it is possible to construct a liquid transfer apparatus for transferring liquid by utilizing the siphon principle from a supply source unit for the liquid in which one end side of the liquid retainer is immersed to a supply destination unit for the liquid in which another end side of the liquid retainer is immersed. Therefore, when the liquid retainer according to the present invention, which has the simple structure using the substantially flat-plate-like three-dimensional knit and the pouch, is used in the liquid transfer apparatus for transferring liquid by utilizing the siphon principle, the structure of the liquid transfer apparatus can be simplified and downsized.

In the present invention, the pouch includes: an inlet side region continuous with an inlet port through which the liquid flows into the pouch; an outlet side region continuous with an outlet port through which the liquid flows out from the pouch; and a connection region connecting the inlet side region and the outlet side region to each other. Further, the three-dimensional knit is arranged at least in the inlet side region and the outlet side region, and the liquid passes through the pouch. In this case, a pump, a heat exchanger, and the like may be connected to the inlet port and the outlet port. According to the present invention, the pressure increase in the pouch can be suppressed by the plurality of elastic chemical-fiber coupling strands. Thus, it is not necessary for the pump, the heat exchanger, and the like which are connected to the liquid retainer to have high pressure resistance.

Further, according to studies by the inventors of the present application, when the three-dimensional knit formed into the substantially flat-plate-like shape is arranged at least in the inlet side region and the outlet side region, the liquid passing through the pouch diffuses substantially uniformly at parts corresponding to the substantially flat-plate-like three-dimensional knit. Thus, with this structure, all the parts corresponding to the substantially flat-plate-like three-dimensional knit in the inlet side region and the outlet side region can be substantially uniformly cooled or warmed.

In the present invention, the three-dimensional knit is arranged also in the connection region. Further, in the present invention, it is preferred that the pouch be formed of the inlet side region, the outlet side region, and the connection region, and that the three-dimensional knit be arranged in substantially all of the inlet side region, the outlet side region, and the connection region.

With this structure, liquid can be retained with a predetermined retention force between the plurality of mesh-like knit garments of the three-dimensional knit arranged substantially over the entire region in the pouch. Thus, even when the liquid retainer is used while standing the pouch, the liquid can be retained between the plurality of mesh-like knit garments substantially over the entire region of the pouch so that the cooling effect or the heating effect can be yielded substantially over the entire pouch. Further, with this structure, when the liquid retainer is used as the cooling pillow for cooling the head of a sleeper, the head laid on the liquid retainer can be supported with the plurality of elastic chemical-fiber coupling strands of the three-dimensional knit arranged substantially over the entire region in the pouch. Thus, the pressure increase in the pouch can be effectively suppressed, and the comfort to the head laid on the liquid retainer can be further improved. Still further, with this structure, the liquid passing through the pouch substantially uniformly diffuses substantially over the entire region in the pouch. Thus, substantially the entire region of the pouch can be substantially uniformly cooled or warmed.

In the present invention, it is preferred that the pouch be formed into the pouch shape by welding outer edges of two films each made of one of a resin and rubber to each other, and that the pouch include a substantially T-shaped welded portion formed by welding the two films to each other, the substantially T-shaped welded portion partitioning the inlet side region, the outlet side region, and the connection region from one another. With this structure, the inlet side region, the outlet side region, and the connection region are partitioned from one another by the substantially T-shaped welded portion formed at the time of welding the two films to each other. Thus, the structure of the liquid retainer can be further simplified.

In the present invention, for example, the pouch is formed into a substantially rectangular shape, and the inlet port and the outlet port are formed on one end side in a longitudinal direction of the pouch formed into the substantially rectangular shape, whereas the connection region is formed on another end side in the longitudinal direction of the pouch. In this case, a long distance can be secured between the pair of the inlet port and the outlet port and the connection region. Therefore, larger areas can be secured for the inlet side region and the outlet side region.

In the present invention, it is preferred that the liquid retainer be formed into a substantially belt-like shape, and further include: a first engagement member fixed to one surface on one end side in a longitudinal direction of the liquid retainer formed into the substantially belt-like shape; and a second engagement member engageable with the first engagement member to detachably connect the one surface on the one end side in the longitudinal direction of the liquid retainer and another surface on another end side in the longitudinal direction of the liquid retainer to each other, the second engagement member being fixed to the another surface on the another end side in the longitudinal direction of the liquid retainer. With this structure, the liquid retainer formed into the substantially belt-like shape can be used while being wrapped around the head, the arm, the leg, and the like of a human body.

In the present invention, the pouch includes: an inlet side region continuous with an inlet port through which the liquid flows into the pouch; an outlet side region continuous with an outlet port through which the liquid flows out from the pouch; and a connection region connecting the inlet side region and the outlet side region to each other, that the three-dimensional knit be arranged in substantially all of the inlet side region, the outlet side region and the connection region, and that the liquid pass through the pouch. With this structure, even under a state in which the liquid retainer is wrapped around the head, the arm, the leg, and the like of a human body, the liquid can be circulated in the pouch.

The liquid retainer according to the present invention may be used for a cooling and heating apparatus including: a pump for supplying the liquid into the liquid retainer while being connected to the liquid retainer; and a heat exchanger for performing one of cooling and heating of the liquid which is supplied to the liquid retainer. This cooling and heating apparatus has a simple structure capable of cooling and warming a part of a human body, predetermined devices, and the like. Further, even when the liquid retainer is used while standing the pouch, the cooling effect or the heating effect can be yielded in all the parts corresponding to the substantially flat-plate-like three-dimensional knit. Further, when the liquid retainer is used as the cooling pillow, the pressure increase in the pouch can be suppressed, and the comfort to the head laid on the liquid retainer can be improved. Further, all the parts corresponding to the substantially flat-plate-like three-dimensional knit in the inlet side region and the outlet side region can be substantially uniformly cooled or warmed.

The liquid retainer according to the present invention may be used for a liquid transfer apparatus including; a supply source unit for the liquid and a supply destination unit for the liquid. In this liquid transfer apparatus the supply source unit and the supply destination unit are arranged so that a liquid level of the supply source unit is higher than a liquid level of the supply destination unit, and the liquid retainer is formed into a substantially belt-like shape. Further, one end side in a longitudinal direction of the liquid retainer formed into the substantially belt-like shape is immersed in the liquid in the supply source unit, and another end side in the longitudinal direction of the liquid retainer is immersed in the liquid in the supply destination unit. Still further, the pouch is opened on the one end side in the longitudinal direction of the liquid retainer and the another end side in the longitudinal direction of the liquid retainer, and the liquid retainer transfers the liquid from the supply source unit into the supply destination unit via a position higher than the liquid level of the supply source unit. This liquid transfer apparatus uses the liquid retainer having the simple structure using the substantially flat-plate-like three-dimensional knit and the pouch. Thus, the structure of the liquid transfer apparatus for transferring liquid by utilizing the siphon principle can be simplified and downsized.

As described above, according to the liquid retainer and the cooling and heating apparatus of the present invention, it is possible to cool and warm a part of a human body, predetermined devices, and the like with a simple structure. Further, when the liquid retainer of the present invention is used for the liquid transfer apparatus for transferring liquid by utilizing the siphon principle, it is possible to simplify and downsize the structure of the liquid transfer apparatus.

### BRIEF DESCRIPTION OF THE DRAWINGS

FIG. 1 is a block diagram of a schematic structure of a cooling and heating apparatus according to an embodiment of the present invention.
FIG. 2 is a plan view of a schematic structure of a head cooling pillow illustrated in FIG. 1.
FIG. 3 is an enlarged sectional view illustrating the structure of the head cooling pillow, which is illustrated in FIG. 2.
FIG. 4 is a plan view of a knit garment of a three-dimensional knit illustrated in FIG. 2.
FIGS. 5A and 5B are each a view illustrating a modification of the three-dimensional knit illustrated in FIG. 3.
FIGS. 6A to 6D are photographs showing temperature-distribution experimental results of the head cooling pillow at a time when cooling water is flowed into the head cooling pillow illustrated in FIG. 2.
FIGS. 7A to 7D are photographs showing temperature-distribution experimental results of a head cooling pillow in a comparative example at a time when cooling water is flowed into the head cooling pillow, the head cooling pillow including a pouch in which the three-dimensional knit is not arranged.
FIG. 8 is a front view of a cooling band according to the embodiment of the present invention.
FIG. 9 is a rear view of the cooling band illustrated in FIG. 8.
FIG. 10 is a view illustrating how to use the cooling band illustrated in FIG. 8.
FIG. 11 is a front view of a cooling pad according to the embodiment of the present invention.
FIGS. 12A and 12B are schematic views of a liquid transfer apparatus according to the embodiment of the present invention; specifically, FIG. 12A is a schematic side view of the liquid transfer apparatus, and FIG. 12B is a view of a liquid transfer tool and a liquid supply source container viewed in a direction of arrows E-E in FIG. 12A.

### DETAILED DESCRIPTION OF THE PREFERRED EMBODIMENTS

In the following, description is made of embodiments of the present invention with reference to the drawings.

### First embodiment

### (Schematic structure of cooling and heating apparatus)

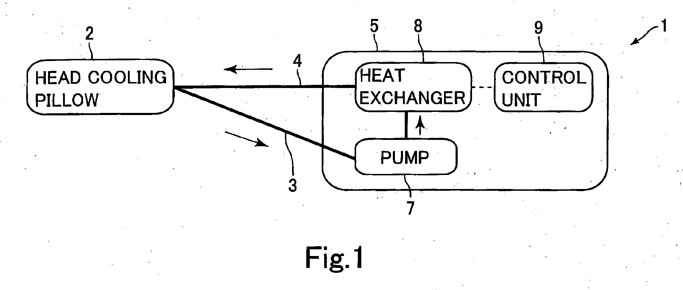

FIG. 1 is a block diagram of a schematic structure of a cooling and heating apparatus 1 according to an embodiment of the present invention.

The cooling and heating apparatus 1 according to this embodiment is a head cooling apparatus for cooling the head of a sleeping user. Therefore, in the following, the cooling and heating apparatus 1 according to this embodiment is also referred to as "head cooling apparatus 1." For example, the head cooling apparatus 1 is used while being set to beds in hospitals, nursing homes, and the like. As illustrated in FIG. 1, the head cooling apparatus 1 includes a head cooling pillow 2 (hereinafter abbreviated as "pillow 2") as a liquid retainer on which the user's head is laid, and a mechanical unit 5 connected to the pillow 2 through intermediation of flexible tubes 3 and 4. Cooling liquid circulates between the pillow 2 and the mechanical unit 5. In this embodiment, the cooling liquid which circulates between the pillow 2 and the mechanical unit 5 is cooling water. Further, for example, the pillow 2 is set on a bed, and the mechanical unit 5 is set to a rail surrounding the bed. Note that, as described below, the pillow 2 is formed into a flattened shape, and hence, normally, used while being overlaid on a general pillow and a cushion. In other words, a height and the like of the pillow 2 on which the user's head is laid are adjusted with the general pillows and cushions.

The tubes 3 and 4 are made of a heat-resistant material which is excellent in heat resistance and hard to break. The mechanical unit 5 includes a pump 7 for supplying the cooling water to the pillow 2, a heat exchanger 8 for cooling the cooling water to be supplied to the pillow 2, and a control unit 9 for controlling the heat exchanger 8. The pump 7 is connected to a cooling-water outlet side of the pillow 2 through intermediation of the tube 3. The heat exchanger 8 is connected to a cooling-water inlet side of the pillow 2 through intermediation of the tube 4. Further, the heat exchanger 8 is connected to a discharge side of the pump 7 through intermediation of a predetermined pipe. The heat exchanger 8 includes thermoelectric elements such as a Peltier element, and cools the cooling water to be supplied to the pillow 2 by the pump 7. The control unit 9 controls the heat exchanger 8 so as to control a temperature of the cooling water which is supplied to the pillow 2.

In this embodiment, the pillow 2, the pump 7, and the heat exchanger 8 are connected to one another without intermediation of a tank for storing the cooling water. In other words, in this embodiment, the pillow 2, the pump 7, and the heat exchanger 8 form a closed loop. Note that, the mechanical unit 5 may further include a small tank for releasing air mixed in the circulating cooling water.

### (Structure of head cooling pillow)

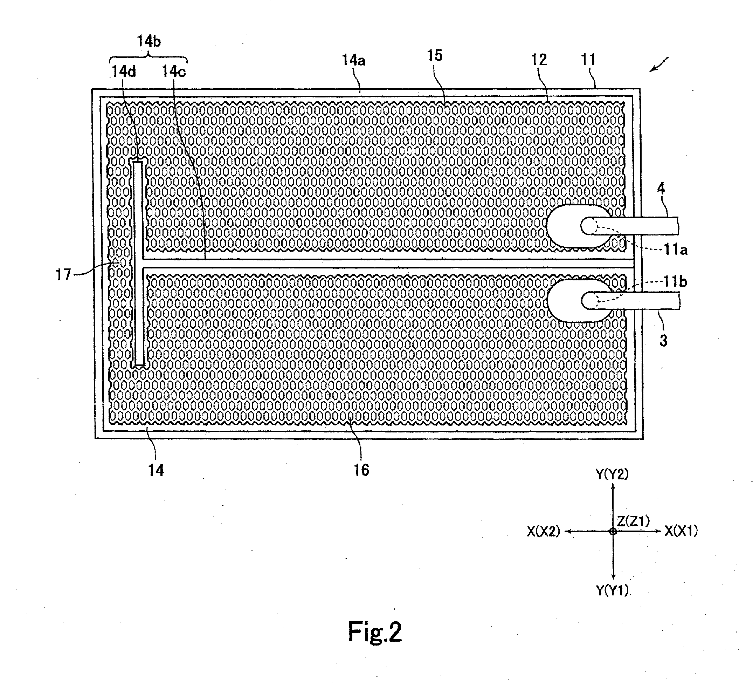

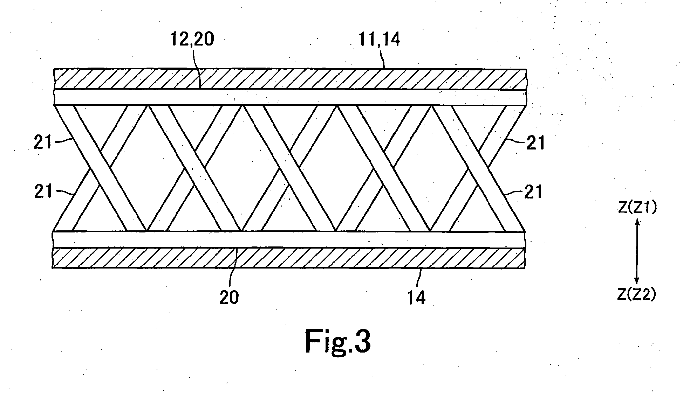

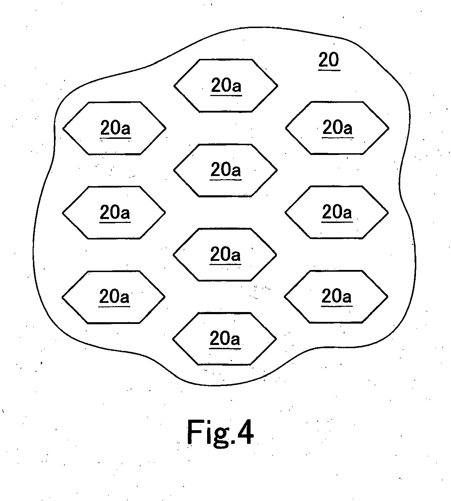

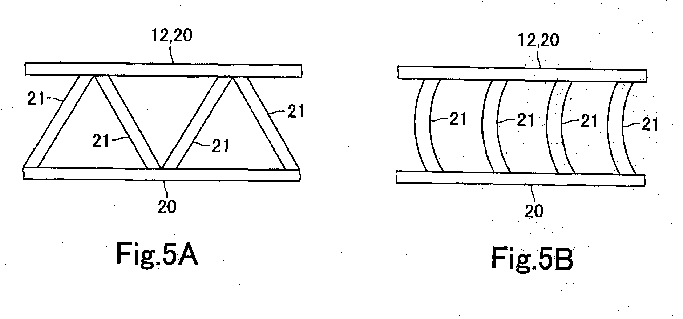

FIG. 2 is a plan view of a schematic structure of the head cooling pillow 2 illustrated in FIG. 1. FIG. 3 is an enlarged sectional view illustrating the structure of the head cooling pillow 2, which is illustrated in FIG. 2. FIG. 4 is a plan view of a knit garment 20 of a three-dimensional knit 12 illustrated in FIG. 2. FIGS. 5A and 5B are each a view illustrating a modification of the three-dimensional knit 12 illustrated in FIG. 3. In the following description of the head cooling pillow 2, three directions orthogonal to one another are respectively referred to as an X-direction, a Y-direction, and a Z-direction, and the X-direction is referred to as a horizontal direction, the Y-direction is referred to as a fore-and-aft direction, and the Z-direction is referred to as an upper-and-lower direction. Further, an X1-direction side is referred to as a "right" side, an X2-direction side is referred to as a "left" side, a Y1-direction side is referred to as a "front" side, a Y2-direction side is referred to as a "rear" side, a Z1-direction side is referred to as an "upper" side, and a Z2-direction side is referred to as a "lower" side.

The pillow 2 is formed into a substantially rectangular-parallelepiped shape flattened in the upper-and-lower direction (direction perpendicular to the drawing sheet of FIG. 2). In other words, the pillow 2 is formed into a substantially rectangular shape so that a longitudinal direction thereof corresponds to the horizontal direction as viewed in the upper-and-lower direction. The pillow 2 includes a pouch 11 formed into a pouch shape so that the cooling water passes therethrough, and the three-dimensional knit 12 arranged in the pouch 11. In this embodiment, the head supported by the pillow 2 abuts against an upper surface of the pillow 2. Further, the horizontal direction substantially corresponds to a width direction of the head supported by the pillow 2. The body trunk of the user whose head is supported by the pillow 2 comes to one side in the fore-and-aft direction. Note that, the pillow 2 may be formed into a substantially oblong shape or a substantially elliptical shape as viewed in the upper-and-lower direction.

The pouch 11 includes two films 14 (refer to FIG. 3): a film 14 forming the upper surface of the pillow 2; and another film 14 forming a lower surface of the pillow 2. The films 14 are each formed into a substantially rectangular shape so that a longitudinal direction thereof corresponds to the horizontal direction. Further, the film 14 is made of a resin or rubber having water blocking properties (properties of preventing liquid permeation) and flexibility, and hence the pouch 11 has the water blocking properties and the flexibility. The film 14 in this embodiment includes a resin film made of a transparent or white resin. The pouch 11 is formed into a pouch shape by welding outer edges of the two films 14 to each other. Along the outer edges of the two films 14, a substantially rectangular frame-like welded portion 14a is formed by welding the two films 14 to each other. Further, the pouch 11 includes a substantially T-shaped welded portion 14b formed by welding the two films 14 to each other.

The welded portion 14b includes a linear first welded portion 14c substantially parallel to the horizontal direction and a linear second welded portion 14d substantially parallel to the fore-and-aft direction. The first welded portion 14c is formed in a range from a right end toward a left end side of the pouch 11. A right end of the first welded portion 14c is continuous with the welded portion 14a. Further, the first welded portion 14c is formed at a substantially central position in the fore-and-aft direction. The second welded portion 14d is formed at a predetermined interval with respect to the left end of the pouch 11. Further, the second welded portion 14d is formed at a predetermined interval with respect to each end in the fore-and-aft direction of the pouch 11. A center in the fore-and-aft direction of the second welded portion 14d is continuous with the left end of the first welded portion 14c.

The pouch 11 includes an inlet port 11a through which the cooling water flows in and an outlet port 11b through which the cooling water flows out. The inlet port 11a and the outlet port 11b are formed on the right end side of the pouch 11. Further, the inlet port 11a and the outlet port 11b are formed in a manner of sandwiching the first welded portion 14c in the fore-and-aft direction. Specifically, the inlet port 11a is formed on the rear side with respect to the first welded portion 14c, and the outlet port 11b is formed on the front side with respect to the first welded portion 14c. Further, the inlet port 11a and the outlet port 11b are formed through the film 14 forming the upper surface of the pillow 2. One end of the tube 4 is connected to the inlet port 11a, and one end of the tube 3 is connected to the outlet port 11b. Note that, not only through the film 14 forming the upper surface of the pillow 2 or instead of the film 14 forming the upper surface of the pillow 2, the inlet port 11a and the outlet port 11b may be formed through the film 14 forming the lower surface of the pillow 2.

In the pouch 11, there are formed an inlet side region 15 continuous with the inlet port 11a, an outlet side region 16 continuous with the outlet port 11b, and a connection region 17 connecting the inlet side region 15 and the outlet side region 16 to each other. In this embodiment, in the pouch 11, the inlet side region 15 includes a region on the rear side with respect to the first welded portion 14c and a region on the left side with respect to a left end of the first welded portion 14c and on the rear side with respect to a rear end of the second welded portion 14d. Further, in the pouch 11, the outlet side region 16 includes a region on the front side with respect to the first welded portion 14c and a region on the left side with respect to the left end of the first welded portion 14c and on the front side with respect to a front end of the second welded portion 14d. In addition, a region between the second welded portion 14d and a left end of the pouch 11 forms the connection region 17.

As described above, the pouch 11 includes therein the inlet side region 15, the outlet side region 16, and the connection region 17. Further, the inlet side region 15, the outlet side region 16, and the connection region 17 are partitioned from one another by the welded portion 14b.

As illustrated in FIG. 3, the three-dimensional knit 12 refers to a three-dimensional fabric structure (three-dimensional knit) including two mesh-like knit garments 20 arranged substantially parallel to each other and a plurality of coupling strands 21 for coupling the two knit garments 20 to each other. The three-dimensional knit 12 is formed into a substantially flat-plate-like shape, and arranged in the pouch 11 so that a thickness direction and the upper-and-lower direction thereof substantially correspond to each other. Further, the three-dimensional knit 12 is flexible.

The knit garment 20 is made of chemical fiber such as polyethylene terephthalate (PET) and polytrimethylen terephthalate (PTT) or natural fiber. As illustrated in FIG. 4, the knit garment 20 includes a plurality of substantially hexagonal opening portions 20a formed at equal intervals, and the plurality of opening portions 20a thus formed constitute the mesh-like shape of the knit garment 20. Note that, the shape of the opening portions 20a is not limited to the substantially hexagonal shape, and may include substantially polygonal shapes such as a substantially quadrangular shape and a substantially octagonal shape, a circular shape, an elliptical shape, or an oblong shape.

The coupling strand 21 is made of elastic chemical fiber. Specifically, the chemical fiber forming the coupling strand 21 includes PET, PTT, polybutylene terephthalate (PBT), and nylon. The plurality of coupling strands 21 are arranged between the two knit garments 20 so that the three-dimensional knit 12 yields elasticity in the thickness direction. For example, as illustrated in FIG. 3, the plurality of coupling strands 21 are arranged between the two knit garments 20 in a manner that the coupling strands 21 intersect with each other in each pair as viewed in the fore-and-aft direction or the horizontal direction.

Note that, the plurality of coupling strands 21 may be arranged between the two knit garments 20 in a manner that the coupling strands 21 intersect with each other in each of the pairs and roots of the coupling strands 21 intersecting with each other in each of the pairs are connected through another coupling strand 21 substantially parallel to the upper-and-lower direction. Alternatively, for example, as illustrated in FIG. 5A, the plurality of coupling strands 21 may be arranged between the two knit garments 20 in a manner that each pair of the coupling strands 21 forms a triangle as viewed in the fore-and-aft direction or the horizontal direction, or as illustrated in FIG. 5B, arranged between the two knit garments 20 in a manner that each of the coupling strands 21 forms a substantially circular-arc shape as viewed in the fore-and-aft direction or the horizontal direction. Still alternatively, the plurality of coupling strands 21 may be arranged between the two knit garments 20 by combining those arrangement methods with each other.

The three-dimensional knit 12 in this embodiment is, for example, a product "FUSION (trademark)" manufactured by Asahi Kasei Fibers Corporation. A specific configuration of the product "FUSION (trademark)" manufactured by Asahi Kasei Fibers Corporation is disclosed in Japanese Patent No. 3394183, Japanese Patent No. 3943011, Japanese Patent No. 4001890, and the like.

The three-dimensional knit 12 is arranged in the inlet side region 15, the outlet side region 16, and the connection region 17. Specifically, one three-dimensional knit 12 is arranged in substantially all of the inlet side region 15, the outlet side region 16, and the connection region 17. In other words, the three-dimensional knit 12 is arranged substantially over the entire region in the pouch 11. As illustrated in FIG. 3, an upper surface and a lower surface of the three-dimensional knit 12 are held in contact with the films 14, and cooling water having a volume substantially equal to a spatial volume of the three-dimensional knit 12 can be stored in the pouch 11. As described above, the pouch 11 and the three-dimensional knit 12 are flexible, and hence the pillow 2 as a whole is also flexible.

In the pillow 2 structured as described above, the cooling water having flowed-in through the inlet port 11a flows out through the outlet port 11b after passing through the inlet side region 15, the connection region 17, and the outlet side region 16 in the stated order. In the pouch 11, the cooling water is retained by the three-dimensional knit 12. Specifically, in the pouch 11, the cooling water is retained between the two knit garments 20.

### (Main effect of this embodiment)

As descried above, in this embodiment, the pillow 2 is formed by arranging the three-dimensional knit 12 having a substantially flat-plate-like shape in the pouch 11. Thus, in this embodiment, the head of a sleeping user can be cooled with a simple structure using the three-dimensional knit 12 having a substantially flat-plate-like shape and the pouch 11. Further, in this embodiment, the pouch 11 is formed into a pouch shape by welding the outer edges of the two films 14 made of a resin or rubber to each other. The inlet side region 15, the outlet side region 16, and the connection region 17 are partitioned from one another by the substantially T-shaped welded portion 14b formed by welding the two films 14 to each other. Thus, in this embodiment, the structure of the pillow 2 can be further simplified.

In this embodiment, the three-dimensional knit 12 is arranged in the inlet side region 15, the outlet side region 16, and the connection region 17, and the head laid on the pillow 2 is supported with the plurality of elastic coupling strands 21. Thus, in this embodiment, a pressure increase in the pouch 11 can be suppressed by the plurality of coupling strands 21, and hence it is not necessary for the pump 7 and the heat exchanger 8 to have high pressure resistance. Further, in this embodiment, the head laid on the pillow 2 is supported with the plurality of elastic coupling strands 21, and hence comfort to the head laid on the pillow 2 can be improved. In particular, in this embodiment, the three-dimensional knit 12 is arranged substantially over the entire region in the pouch 11, and hence the pressure increase in the pouch 11 can be more effectively suppressed. Further, the comfort to the head laid on the pillow 2 can be further improved. In addition, in this embodiment, the head laid on the pillow 2 is supported with the plurality of coupling strands 21, and hence the cooling water can be secured and allowed to pass under the head even when the head is laid on the pillow 2. Thus, in this embodiment, the head can be appropriately cooled.

In this embodiment, the inlet port 11a and the outlet port 11b are formed on the right end side of the pouch 11, and the connection region 17 is formed on the left end side of the pouch 11. Thus, in this embodiment, larger areas can be secured for the inlet side region 15 and the outlet side region 16.

In this embodiment, the pouch 11 includes therein the inlet side region 15, the outlet side region 16, and the connection region 17. Thus, the cooling water having flowed-in through the inlet port 11a is prevented from immediately flowing out through the outlet port 11b. Thus, in this embodiment, the cooling water passing through the pouch 11 can be diffused substantially over the entire region in the pouch 11. Further, the cooling water can be prevented from stagnating in the pouch 11. In particular, in this embodiment, the three-dimensional knit 12 is arranged in all of the inlet side region 15, the outlet side region 16, and the connection region 17. According to studies by the inventors of the present application, the cooling water passing through the pouch 11 substantially uniformly diffuses substantially over the entire region in the pouch 11. Thus, in this embodiment, substantially the entire region of the pouch 11 can be substantially uniformly cooled. In the following, more specific description is made of this effect based on experimental results.

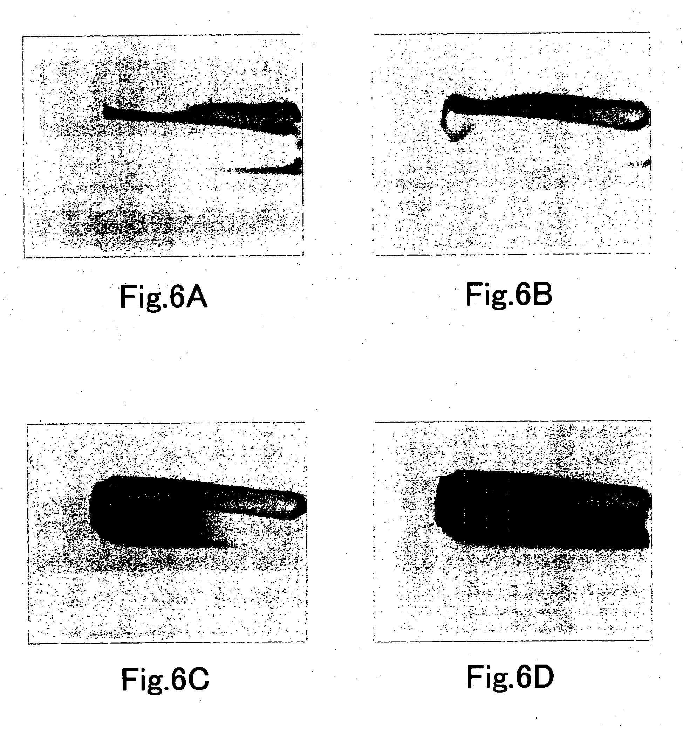

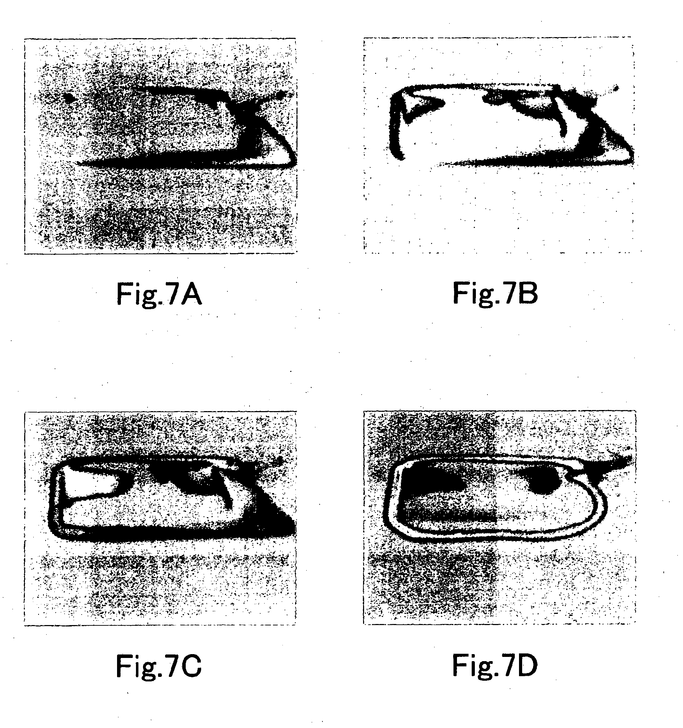

FIGS. 6A to 6D are photographs showing temperature-distribution experimental results of the head cooling pillow 2 at a time when the cooling water is flowed into the head cooling pillow 2 illustrated in FIG. 2. FIGS. 7A to 7D are photographs showing temperature-distribution experimental results of a head cooling pillow in a comparative example at a time when cooling water is flowed into the head cooling pillow, the head cooling pillow including a pouch 11 in which the three-dimensional knit 12 is not arranged. Note that, in FIGS. 6A to 7D, blackish regions correspond to regions of low temperature, and in FIGS. 7A to 7D, whitish regions in the blackish regions correspond to regions of much lower temperature. Further, in FIGS. 6A to 7D, regions of substantially the equal temperatures are indicated by the same colors. Further, in the experiment, a product "FUSION (trademark)" under model number "AKE64030," which is manufactured by Asahi Kasei Fibers Corporation, was used as the three-dimensional knit 12.

In the experiment, a pillow 2 under a state in which cooling water was not contained therein was arranged in a manner that a thickness direction (Z-direction) thereof corresponded to a vertical direction, and then cooling water was flowed-in through the inlet port 11a. The cooling water having thus flowed-in through the inlet port 11a first flowed through the inlet side region 15 while diffusing over a part of the entire three-dimensional knit 12 in the inlet side region 15. Thus, as shown in FIGS. 6A and 6B, a temperature of substantially the entire inlet side region 15 gradually decreased substantially uniformly from a point near the inlet port 11a.

After that, the cooling water flowed sequentially through the connection region 17 and the outlet side region 16 while diffusing over parts of the entire three-dimensional knit 12 in the connection region 17 and the outlet side region 16. Thus, as shown in FIGS. 6C and 6D, a temperature gradually and substantially uniformly decreased in substantially all of the connection region 17 and the outlet side region 16. Finally, a temperature substantially uniformly decreased in substantially all of the inlet side region 15, the outlet side region 16, and the connection region 17. As described above, in the pillow 2 in this embodiment, the cooling water passing through the pouch 11 can substantially uniformly cool substantially the entire region in the pouch 11 by substantially uniformly diffusing substantially over the entire region in the pouch 11.

Note that, FIG. 6A shows a state two minutes and ten seconds after a start of inflow of the cooling water into the pillow 2, FIG. 6B shows a state three minutes and ten seconds after the start of the inflow of the cooling water into the pillow 2, FIG. 6C shows a state four minutes and forty seconds after the start of the inflow of the cooling water into the pillow 2, and FIG. 6D shows a state five minutes and twenty seconds after the start of the inflow of the cooling water into the pillow 2.

Further, in a comparative experiment, the pillow including the pouch 11, in which the three-dimensional knit 12 was not arranged, was used as a comparative example. The pillow under a state in which cooling water was not contained therein was arranged in a manner that a thickness direction thereof corresponded to the vertical direction, and then cooling water was flowed-in through the inlet port 11a. In the pillow, the cooling water having thus flowed-in through the inlet port 11a flowed along outer edges of the pillow, at which the cooling water was easy to flow. Thus, as shown in FIGS. 7A to 7D, a temperature at the outer edges of the pillow decreased, while a temperature on a center side of the pillow did not decrease. As described above, in the pillow including the pouch 11 in which the three-dimensional knit 12 is not arranged therein, substantially the entire region of the pouch 11 cannot be substantially uniformly cooled.

Note that, FIG. 7A shows a state twenty seconds after a start of inflow of the cooling water into the pillow, FIG. 7B shows a state forty seconds after the start of the inflow of the cooling water into the pillow, FIG. 7C shows a state one minute and thirty seconds after the start of the inflow of the cooling water into the pillow, and FIG. 7D shows a state nine minutes and forty seconds after the start of the inflow of the cooling water into the pillow.

### (Modifications of head cooling pillow)

In the above-mentioned embodiment, the three-dimensional knit 12 is arranged substantially over the entire region of the inlet side region 15, but the three-dimensional knit 12 may be arranged over a part of the inlet side region 15. Further, the three-dimensional knit 12 is arranged substantially over the entire region of the outlet side region 16, but the three-dimensional knit 12 may be arranged over a part of the outlet side region 16. Still further, the three-dimensional knit 12 is arranged substantially over the entire region of the connection region 17, but the three-dimensional knit 12 may be arranged over a part of the connection region 17.

In the above-mentioned embodiment, the three-dimensional knit 12 is arranged in the inlet side region 15, the outlet side region 16, and the connection region 17, but the three-dimensional knit 12 may be arranged only in the inlet side region 15 and the outlet side region 16 except the connection region 17. In this case, the cooling water passing through the pouch 11 diffuses substantially uniformly at parts corresponding to the three-dimensional knit 12 in the inlet side region 15 and the outlet side region 16. Thus, in this case, all the parts corresponding to the three-dimensional knit 12 in the inlet side region 15 and the outlet side region 16 can be substantially uniformly cooled.

In the above-mentioned embodiment, the one three-dimensional knit 12 is arranged in the pouch 11, but a plurality of three-dimensional knits 12 divided from one another in the fore-and-aft direction and the horizontal direction may be arranged in the pouch 11. Alternatively, two or more three-dimensional knits 12 laminated on each other in the upper-and-lower direction may be arranged in the pouch 11.

### (First modification of liquid retainer)

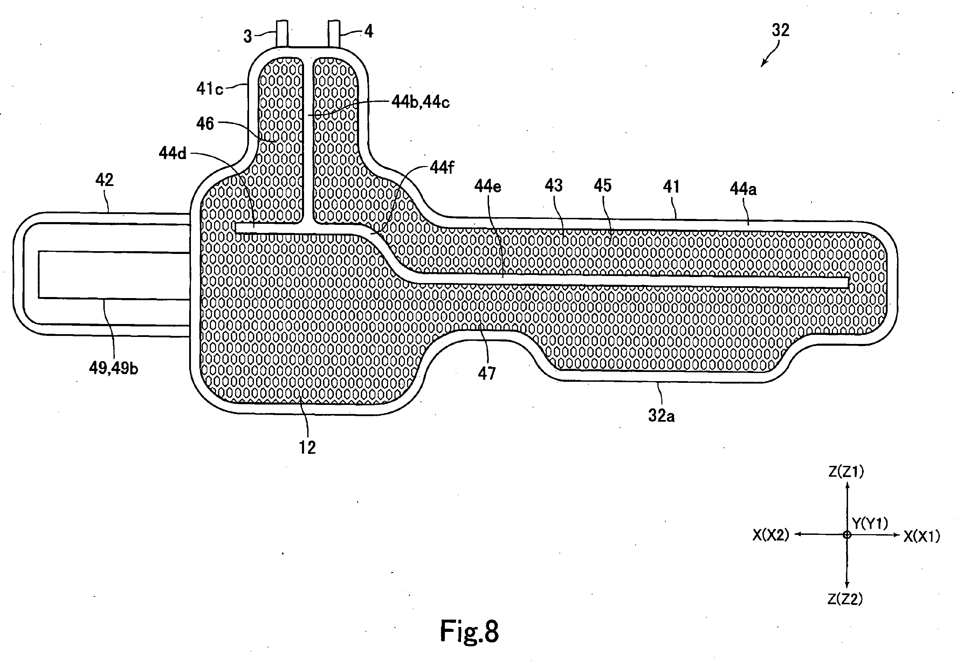

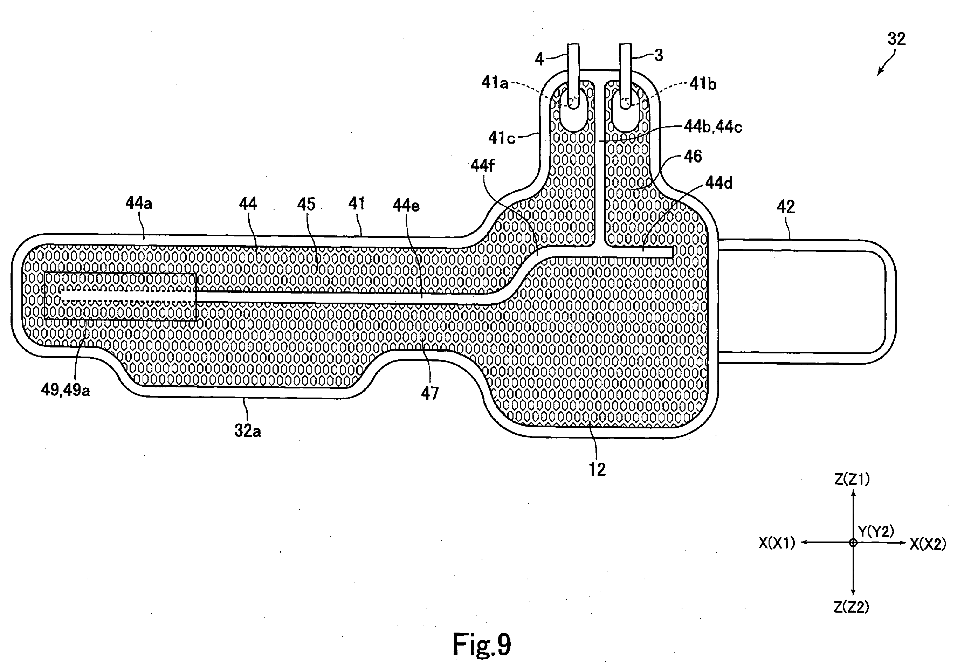

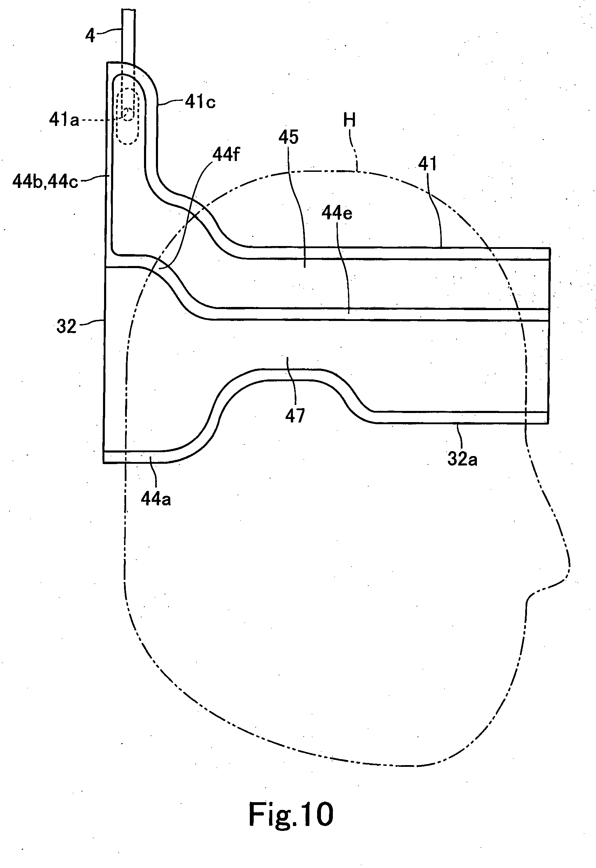

FIG. 8 is a front view of a cooling band 32 according to the embodiment of the present invention. FIG. 9 is a rear view of the cooling band 32 illustrated in FIG. 8. FIG. 10 is a view illustrating how to use the cooling band 32 illustrated in FIG. 8.

In the above-mentioned embodiment, the liquid retainer includes the pillow 2 for cooling the head of a sleeping user, but the liquid retainer according to the present invention may include the belt-like cooling band 32 used while being wrapped around the head of a user for cooling the head of the user. In the following, description is made of a structure of the cooling band 32.

Note that, in the following description of the cooling band 32, components of the cooling band 32, which are the same as those of the pillow 2, are denoted by the same reference symbols as those in the above-mentioned embodiment, and description thereof is omitted or simplified. Further, in the following description of the cooling band 32, in FIGS. 8 and 9, the X-direction is referred to as a horizontal direction, the Y-direction is referred to as a fore-and-aft direction, and the Z-direction is referred to as an upper-and-lower direction. Further, in FIGS. 8 and 9, the X1-direction side is referred to as a "right" side, the X2-direction side is referred to as a "left" side, the Y1-direction side is referred to as a "front" side, the Y2-direction side is referred to as a "rear" side, the Z1-direction side is referred to as an "upper" side, and the Z2-direction side is referred to as a "lower" side.

The cooling band 32 is formed into a substantially belt-like shape elongated in the horizontal direction. The cooling band 32 includes a substantially belt-like pouch 41 formed into a pouch shape so that cooling water passes therethrough, the three-dimensional knit 12 arranged in the pouch 41, and a belt-like band portion 42 connected to the pouch 41. The band portion 42 is formed to be connected to a left end of the pouch 41.

The pouch 41 includes two films 43 and 44: a film 43 forming a front surface of the cooling band 32; and a film 44 forming a rear surface of the cooling band 32. The films 43 and 44 are each formed into a substantially belt-like shape so that a longitudinal direction thereof corresponds to the horizontal direction. On a left end side of the films 43 and 44, a vertical width of each of the films 43 and 44 increases toward both sides in the upper-and-lower direction. Further, in a predetermined range from a substantially central position in the horizontal direction of each of the films 43 and 44 toward a right end side, the vertical width of each of the films 43 and 44 increases toward the lower side. Still further, the films 43 and 44 are each made of the same material as that for the above-mentioned film 14. The pouch 41 is formed into a pouch shape by welding outer edges of the two films 43 and 44 to each other. Along the outer edges of the two films 43 and 44, a frame-like welded portion 44a is formed by welding the two films 43 and 44 to each other. Further, the pouch 41 includes a substantially T-shaped welded portion 44b formed by welding the two films 43 and 44 to each other.

Note that, an upper end side of a part on the left end side of each of the films 43 and 44, on which the vertical width increases toward both the sides in the upper-and-lower direction, is projected to the upper side. In other words, a projecting portion 41c projected to the upper side is formed on the left end side of the pouch 41. Further, a part in the predetermined range from the substantially central position in the horizontal direction of each of the films 43 and 44 toward the right end side, at which the vertical width of each of the films 43 and 44 increases toward the lower side, comes to the forehead side when the cooling band 32 is wrapped around a head H (refer to FIG. 10) as described below. In other words, this part of the cooling band 32 forms a forehead contact portion 32a which is held in contact with the forehead.

The welded portion 44b includes a linear first welded portion 44c substantially parallel to the upper-and-lower direction, two linear second welded portions 44d and 44e substantially parallel to the horizontal direction, and a curved third welded portion 44f connecting the second welded portion 44d and the second welded portion 44e to each other. The first welded portion 44c is formed on the left end side of the pouch 41 in a manner of passing substantially through the center in the horizontal direction of the projecting portion 41c. The first welded portion 44c is formed in a range from an upper end toward a lower end side of the pouch 41. An upper end of the first welded portion 44c is continuous with the welded portion 44a. The second welded portion 44d is formed at a predetermined interval with respect to the left end of the pouch 41. Further, the second welded portion 44d is formed at a predetermined interval with respect to each end in the upper-and-lower direction of the pouch 41. A center in the horizontal direction of the second welded portion 44d is continuous with a lower end of the first welded portion 44c. The second welded portion 44e is formed at a predetermined interval with respect to a right end of the pouch 41. Still further, the second welded portion 44e is formed at a predetermined interval with respect to each end in the upper-and-lower direction of the pouch 41. The third welded portion 44f is formed in a manner of connecting a right end of the second welded portion 44d and a left end of the second welded portion 44e to each other.

The pouch 41 includes an inlet port 41a through which the cooling water flows in and an outlet port 41b through which the cooling water flows out. The inlet port 41a and the outlet port 41b are formed on the upper end side of the projecting portion 41C. Further, the inlet port 41a and the outlet port 41b are formed in a manner of sandwiching the first welded portion 44c in the horizontal direction. Further, the inlet port 41a and the outlet port 41b are formed through the film 44. One end of the tube 4 is connected to the inlet port 41a, and one end of the tube 3 is connected to the outlet port 41b.

In the pouch 41, there are formed an inlet side region 45 continuous with the inlet port 41a, an outlet side region 46 continuous with the outlet port 41b, and a connection region 47 connecting the inlet side region 45 and the outlet side region 46 to each other. In this embodiment, in the pouch 41, the inlet side region 45 includes a region on the right side with respect to the first welded portion 44c and on the upper side with respect to the second welded portions 44d and 44e and the third welded portion 44f. Further, in the pouch 41, the outlet side region 46 includes a region on the left side with respect to the first welded portion 44c and on the upper side with respect to the second welded portion 44d. Further, in the pouch 41, the connection region 47 includes a region on the lower side with respect to the second welded portions 44d and 44e and the third welded portion 44f. As described above, the pouch 41 includes therein the inlet side region 45, the outlet side region 46, and the connection region 47. The inlet side region 45, the outlet side region 46, and the connection region 47 are partitioned from one another by the welded portion 44b.

The three-dimensional knit 12 is arranged in the inlet side region 45, the outlet side region 46, and the connection region 47 in a manner that a thickness direction and the fore-and-aft direction thereof substantially correspond to each other. Further, the three-dimensional knit 12 is arranged substantially over the entire region in the pouch 41. A front surface and a rear surface of the three-dimensional knit 12 are held in touch with the films 43 and 44, and cooling water having a volume substantially equal to a spatial volume of the three-dimensional knit 12 can be stored in the pouch 41. Note that, similarly to the pillow 2, the cooling band 32 is flexible.

The right end side of the pouch 41 and the band portion 42 are connected to each other with a hook-and-loop fastener 49. A hook member 49a as a first engagement member of the hook-and-loop fastener 49 is fixed to a rear surface on the right end side of the pouch 41, and a loop member 49b as a second engagement member of the hook-and-loop fastener 49 is fixed to a front surface of the band portion 42. The hook member 49a is provided with a hook surface on which hooks are raised, and the loop member 49b is provided with a loop surface on which loops are raised.

As illustrated in FIG. 10, the cooling band 32 structured as described above is used while being wrapped around the head H. In this embodiment, the cooling band 32 is wrapped around the head H in a manner that the rear surface (film 44 side) of the cooling band 32 is held in contact with the head H. Further, the cooling band 32 is wrapped around the head H also in a manner that the inlet port 41a and the outlet port 41b come to the back side of the head and the forehead contact portion 32a of the cooling band 32 comes to the forehead side. In the cooling band 32 wrapped around the head H, the cooling water having flowed-in through the inlet port 41a flows out through the outlet port 41b after passing through the inlet side region 45, the connection region 47, and the outlet side region 46 in the stated order. In the pouch 41, the cooling water is retained by the three-dimensional knit 12.

The cooling band 32 is used while being wrapped around the head H, and hence the cooling band 32 may be used under a state in which the thickness direction of the three-dimensional knit 12 is inclined with respect to the vertical direction. However, as described above, the two mesh-like knit garments 20 are coupled to each other by the plurality of elastic coupling strands 21. Thus, the cooling water can be retained between the two knit garments 20 with a predetermined retention force by utilizing surface tension of the cooling water. Therefore, even when the cooling band 32 is used under the state in which the thickness direction of the three-dimensional knit 12 is inclined with respect to the vertical direction, liquid can be retained between the two knit garments 20 of the three-dimensional knit 12 arranged in the pouch 41 so that the cooling water can be prevented from pooling on a lower side of the pouch 41. As a result, even when the cooling band 32 is used under the state in which the thickness direction of the three-dimensional knit 12 is inclined with respect to the vertical direction, a cooling effect can be yielded over the entire cooling band 32, and hence the head H can be effectively cooled.

Note that, in the cooling band 32, the three-dimensional knit 12 is not arranged in the band portion 42, but the three-dimensional knit 12 may be arranged in the band portion 42. Further, the right end side of the pouch 41 and the band portion 42 may be connected to each other not with the hook-and-loop fastener 49 but with a single hook or the like. Further, through adjustment of a length and a width of the cooling band 32, the cooling band 32 can be used while being wrapped around not only the head H but also around the trunk, the arm, the leg, and the like of a human body.

### (Second modification of liquid retainer)

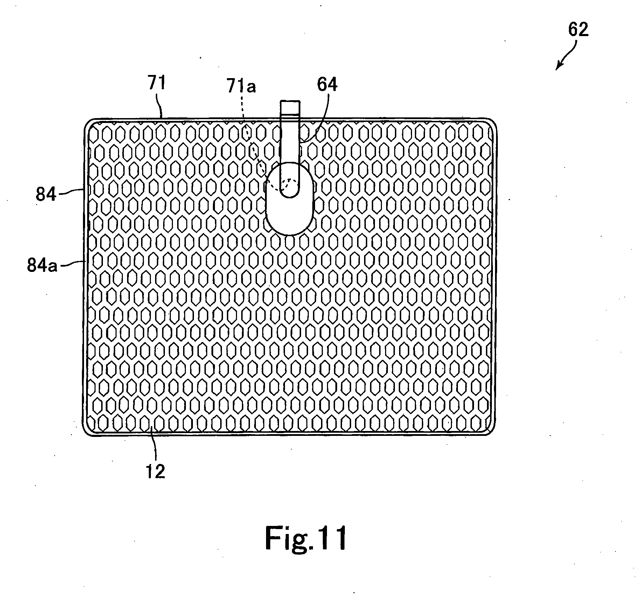

FIG. 11 is a front view of a cooling pad 62 according to the embodiment of the present invention.

In the above-mentioned embodiment, the liquid retainer includes the pillow 2 for cooling the head of a sleeping user, but the liquid retainer according to the present invention may include the cooling pad 62 for cooling the forehead, the arm, the leg, and the like of a human body. In the following, description is made of the structure of the cooling pad 62. Note that, in the following description of the cooling pad 62, components of the cooling pad 62, which are the same as those of the pillow 2, are denoted by the same reference symbols as those in the above-mentioned embodiment, and description thereof is omitted or simplified.

The cooling pad 62 is formed into a substantially square shape or a substantially rectangular shape, and includes a pouch 71 formed into a pouch shape so that cooling water is retained therein, and the three-dimensional knit 12 arranged in the pouch 71. The pouch 71 is formed into a pouch shape by welding outer edges of two films 84 each made of the same material as that for the film 14. Along the outer edges of the two films 84, a frame-like welded portion 84a is formed by welding the two films 84 to each other. Further, the pouch 71 includes an injection port 71a for injecting cooling water into the pouch 71. One end of a tube 64 is connected to the injection port 71a.

The three-dimensional knit 12 is arranged in the pouch 71 in a manner that a thickness direction thereof and a thickness direction of the films 84 substantially correspond to each other. Surfaces of the three-dimensional knit 12 are held in touch with the films 84, and cooling water having a volume substantially equal to a spatial volume of the three-dimensional knit 12 can be stored in the pouch 71. Note that, similarly to the pillow 2, the cooling pad 62 is flexible.

In the cooling pad 62, after the cooling water is injected into the pouch 71 through the tube 64, another end of the tube 64 is sealed by being flattened so as to prevent leakage of the cooling water from the inside of the pouch 71. In the pouch 71, the cooling water is retained by the three-dimensional knit 12.

The cooling pad 62 may be used while being held in contact with the arm, the leg, and the like, and hence the cooling pad 62 may be used under the state in which the thickness direction of the three-dimensional knit 12 is inclined with respect to the vertical direction. However, as described above, the two mesh-like knit garments 20 are coupled to each other by the plurality of elastic coupling strands 21. Thus, the cooling water can be retained between the two knit garments 20 with a predetermined retention force by utilizing surface tension of the cooling water. Therefore, even when the cooling pad 62 is used under the state in which the thickness direction of the three-dimensional knit 12 is inclined with respect to the vertical direction, liquid can be retained between the two knit garments 20 of the three-dimensional knit 12 arranged in the pouch 71 so that the cooling water can be prevented from pooling on a lower side of the pouch 71. As a result, even when the cooling pad 62 is used under the state in which the thickness direction of the three-dimensional knit 12 is inclined with respect to the vertical direction, a cooling effect can be yielded over the entire cooling pad 62, and hence the arm, the leg, and the like can be effectively cooled.

Note that, it is not necessary for the tube 64 to be connected to the injection port 71a as long as the injection port 71a is closed after the cooling water is injected into the pouch 71 through the injection port 71a. Further, through adjustment of a size of the cooling pad 62, the cooling pad 62 can be used also, for example, as an emergency cooling sheet.

### (Other modifications of first embodiment)

In the above-mentioned embodiment, the liquid retainer includes the pillow 2 for cooling the head of a sleeping user, but the liquid retainer according to the present invention may include a mat, a blanket, or a bed sheet for cooling the entire human body other than the head. In this case, it suffices that the liquid retainer is constructed by enlarging the pillow 2 as it is. Further, the liquid retainer according to the present invention may include a jacket or a vest for cooling the trunk of a human body. In this case, it suffices that the pouch 11 of the pillow 2 is formed in conformity with the trunk of a human body.

Still further, the liquid retainer according to the present invention may include a heating pillow, a heating band, a heating pad, a heating sheet, a heating mat, a heating blanket, a heating bed sheet, a heating jacket, or a heating vest for heating a part of a human body. In this case, heating liquid (for example, hot water) passes, for example, through the pouch 11 or 41. Further, in this case, the heating liquid (for example, hot water) is retained in, for example, the pouch 71.

Yet further, the liquid retainer according to the present invention may be configured to cool and heat, for example, animals other than humans. Yet further, the liquid retainer according to the present invention may be configured to cool and heat machines, structures, and the like. For example, a liquid retainer formed into a sheet-like shape may be set on a rear side of a traffic mirror installed on the roadside so as to heat the traffic mirror. Accordingly, fogging and icing on a surface of a traffic mirror used in cold districts or the like can be prevented. Alternatively, the liquid retainer formed into a sheet-like shape may be put, for example, on a windshield of an automobile used in cold districts so as to heat the windshield. Accordingly, ice adhering to the windshield can be melted. Still alternatively, the liquid retainer formed into a sheet-like shape may be installed, for example, on a roof of a house in regions of high snowfall so as to heat the roof at the time of snowfall or snow accumulation. Accordingly, snow accumulated on the roof can be slid off from the roof.

Yet further, the liquid retainer according to the present invention may be configured to cool and heat agricultural products. For example, the liquid retainer formed into a sheet-like shape may be set in a storage box for agricultural products for which temperature control is required so as to cool and heat the agricultural products.

In the above-mentioned embodiment, the cooling water having flowed-in through the inlet port 11a flows out through the outlet port 11b. In addition, as for the pillow 2, the cooling water circulates therein. Alternatively, for example, the inlet port 11a and the outlet port 11b may be sealed after the cooling water is injected into the pouch 11. In other words, it is not necessary for the cooling water to circulate in the pillow 2. In this case, it is not necessary for the pouch 11 to include the welded portion 14b. In still other words, it is not necessary for the pouch 11 to include therein the inlet side region 15, the outlet side region 16, and the connection region 17. Similarly, in the cooling band 32 in which the cooling water having flowed-in through the inlet port 41a flows out through the outlet port 41b, the inlet port 41a and the outlet port 41b may be sealed after the cooling water is injected into the pouch 41 so that the cooling water is not circulated. Further, in the cooling pad 62 in which the cooling water is not circulated, the cooling water may be circulated. Consequently, in the liquid retainer according to the present invention, it is not necessary for the cooling liquid or the heating liquid to be circulated.

### Second embodiment

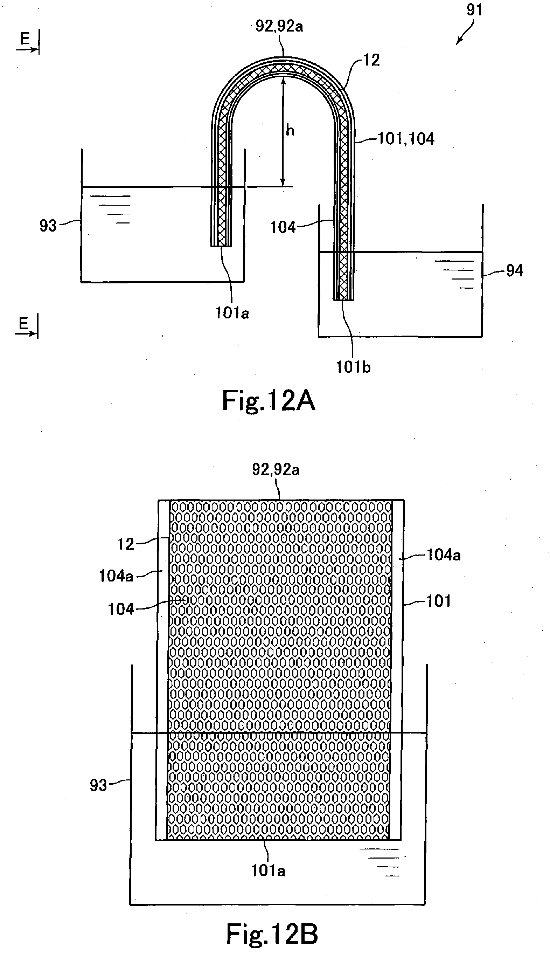

FIGS. 12A and 12B are schematic views of a liquid transfer apparatus 91 according to an embodiment of the present invention. Specifically, FIG. 12A is a schematic side view of the liquid transfer apparatus 91, and FIG. 12B is a view of a liquid transfer tool 92 and a liquid supply source container 93 viewed in a direction of arrows E-E in FIG. 12A.

The liquid transfer apparatus 91 according to this embodiment is an apparatus for transferring liquid by utilizing a siphon principle. The liquid transferred by the liquid transfer apparatus 91 includes ink for ink jet printers. In this case, the liquid transfer apparatus 91 is used while being mounted to the ink jet printer. Further, the liquid transferred by the liquid transfer apparatus 91 includes fertilizer (liquid fertilizer), solvents, oil, chemicals, medicines, blood, or blood isolates. In those cases, the liquid transfer apparatus 91 is used while being mounted to various apparatuses and devices.

The liquid transfer apparatus 91 includes the liquid transfer tool 92 as the liquid retainer, the liquid supply source container 93 as a liquid supply source unit, and a liquid supply destination container 94 as a liquid supply destination unit. Note that, in the following description of the liquid transfer tool 92, components of the liquid transfer tool 92, which are the same as those of the pillow 2, are denoted by the same reference symbols as those in the above-mentioned embodiment, and description thereof is omitted or simplified.

The liquid transfer tool 92 is formed into a substantially rectangular belt-like shape. The liquid transfer tool 92 includes a pouch 101 formed into a pouch shape so that cooling water passes therethrough, and the three-dimensional knit 12 arranged in the pouch 101. The pouch 101 is obtained by forming two films 104, which are made of the same material as that for the film 14, into a substantially rectangular belt-like shape. Further, the pouch 101 is formed into a pouch shape by welding both ends of the two films 104 to each other in a lateral direction of the pouch 101 formed into the belt-like shape. At both ends in the lateral direction of the pouch 101, there are formed linear welded portions 104a along which the two films 104 are welded to each other. Further, both ends 101a and 101b in a longitudinal direction of the pouch 101 are opened.

The three-dimensional knit 12 is arranged in the pouch 101 in a manner that a thickness direction of the three-dimensional knit 12 and the thickness direction of the films 104 substantially correspond to each other. Specifically, the three-dimensional knit 12 is arranged substantially over the entire region in the pouch 101. Surfaces of the three-dimensional knit 12 are held in touch with the films 104, and cooling water having a volume substantially equal to a spatial volume of the three-dimensional knit 12 can be stored in the pouch 101. Note that, similarly to the pillow 2, the liquid transfer tool 92 is flexible.

The liquid supply source container 93 and the liquid supply destination container 94 are arranged so that a liquid level in the liquid supply source container 93 is higher than a liquid level in the liquid supply destination container 94. One end side in a longitudinal direction of the liquid transfer tool 92 (one end 101a side of the pouch 101) is immersed in a liquid in the liquid supply source container 93. Another end side in the longitudinal direction of the liquid transfer tool 92 (another end 101b side of the pouch 101) is immersed in a liquid in the liquid supply destination container 94.

The liquid transfer tool 92 is set under a state of being curved, for example, in a substantially U-shape. Further, the liquid transfer tool 92 is set so that a curved portion 92a comes to an upper side and both the ends in the longitudinal direction of the liquid transfer tool 92 come to a lower side. A level difference h between the liquid level in the liquid supply source container 93 and the curved portion 92a is set, for example, approximately to 500 mm.

As described above, the two mesh-like knit garments 20 are coupled to each other by the plurality of elastic coupling strands 21. Thus, in the liquid transfer tool 92, liquid can be retained between the two knit garments 20 with a predetermined retention force by utilizing surface tension of the liquid. Thus, even when the liquid transfer tool 92 is set so that the curved portion 92a comes to the upper side and both the ends in the longitudinal direction of the liquid transfer tool 92 come to the lower side, the liquid can be retained between the two knit garments 20 of the three-dimensional knit 12 arranged in the pouch 101. As a result, in the liquid transfer apparatus 91, with use of the liquid transfer tool 92 including the pouch 101 having both the opened ends 101a and 101b, the liquid can be transferred, through the curved portion 92a arranged at the position higher than the liquid level of the liquid supply source container 93, from the liquid supply source container 93 in which the one end side of the liquid transfer tool 92 is immersed into the liquid supply destination container 94 in which the another end side of the liquid transfer tool 92 is immersed. In other words, in the liquid transfer apparatus 91, even when a pump or the like for transferring liquid from the liquid supply source container 93 into the liquid supply destination container 94 is not set, the liquid can be transferred from the liquid supply source container 93 into the liquid supply destination container 94 by utilizing the siphon principle using the liquid transfer tool 92.

As described above, in this embodiment, the liquid transfer apparatus 91 is formed by using the liquid transfer tool 92 having a simple structure using the three-dimensional knit 12 having a substantially flat-plate-like shape and the pouch 101. Thus, the structure of the liquid transfer apparatus 91 which utilizes the siphon principle can be simplified and downsized.

## Claims

1. A liquid retainer, comprising:
a pouch (11; 41; 71) having water blocking properties and flexibility and formed into a pouch shape; and
a three-dimensional knit (12):
wherein the pouch (11; 41; 71) is formed into the pouch shape by welding outer edges of two films (14; 43, 44; 84) each made of one of resin and rubber to each other,
wherein the pouch (11; 41; 71) comprises:
an inlet side region (15; 45) continuous with an inlet port (11a; 41a) through which the liquid flows into the pouch (11; 41; 71);
an outlet side region (16; 46) continuous with an outlet port (11b; 41b) through which the liquid flows out from the pouch (11; 41; 71); and
a connection region (17; 47) connecting the inlet side region (15; 45) and the outlet side region (16; 46) to each other,
wherein the pouch (11; 41; 71) comprises a welded portion (14b; 44b) formed by welding the two films (14; 43, 44; 84) to each other, the welded portion (14b; 44b) partitioning the inlet side region (15; 45), the outlet side region (16; 46), and the connection region (17; 47) from one another,
wherein the three-dimensional knit (12) is formed into a substantially flat-plate-like shape and arranged in substantially all of the inlet side region (15; 45), the outlet side region (16; 46), and the connection region (17; 47) in the pouch (11; 41; 71),
wherein the liquid retained by the three-dimensional knit (12) in the pouch (11; 41; 71) comprises one of cooling liquid and heating liquid, and
wherein the liquid passes through the inlet side region (15; 45), the outlet side region (16; 46), and the connection region (17; 47) in the pouch (11; 41; 71) where the three-dimensional knit (12) is arranged
**characterized in that** the three-dimensional knit (12) comprises:
a plurality ofmesh-like knit garments (20) arranged substantially parallel to eachother; and
a plurality ofcoupling strands (21) for coupling the plurality of mesh-like knit garments (20) to each other,
wherein the plurality of coupling strands (21) are made of elastic chemical fiber.

2. A liquid retainer according to claim 1, wherein the pouch (11; 41; 71) is formed of the inlet side region (15; 45), the outlet side region (16; 46), and the connection region (17; 47).

3. A liquid retainer according to claim 1 or 2, wherein the welded portion (14b; 44b) is formed into a substantially T-shape.

4. A liquid retainer according to any one of claims 1 to 3,
wherein the pouch (11; 41; 71) is formed into a substantially rectangular shape,
wherein the inlet port (11a; 41a) and the outlet port (11b; 41b) are formed on one end side in a longitudinal direction of the pouch (11; 41; 71) formed into the substantially rectangular shape, and
wherein the connection region (17; 47) is formed on another end side in the longitudinal direction of the pouch (11; 41; 71).

5. A liquid retainer according to claim1 or 2, wherein the liquid retainer is formed into a substantially belt-like shape, and further comprises:
a first engagement member (49a) fixed to one surface on one end side in a longitudinal direction of the liquid retainer formed into the substantially belt-like shape; and
a second engagement member (49b) engagable with the first engagement member (49a) to detachably connect the one surface on the one end side in the longitudinal direction of the liquid retainer and another surface on another end side in the longitudinal direction of the liquid retainer to each other, the second engagement member (49b) being fixed to the another surface on the another end side in the longitudinal direction of the liquid retainer.

6. A cooling and heating apparatus, comprising:
the liquid retainer according to any one of claims 1 to 5;
a pump (7) for supplying the liquid into the liquid retainer while being connected to the liquid retainer; and
a heat exchanger (8) for performing one of cooling and heating of the liquid which is supplied to the liquid retainer.

## Patentansprüche

1. Flüssigkeitsrückhalteeinrichtung, aufweisend:
einen Beutel (11; 41; 71), der Wasser blockierende Eigenschaften und Flexibilität aufweist und in einer Beutelform ausgebildet ist; und
ein dreidimensionales Gewirk (12),
wobei der Beutel (11; 41; 71) durch Verschweißen von äußeren Rändern von zwei Folien (14; 43, 44; 84), die jeweils aus Harz- oder Gummimaterial hergestellt sind, miteinander in der Beutelform ausgebildet sind,
wobei der Beutel (11; 41; 71) aufweist:
einen Eintrittsseitenbereich (15; 45), der kontinuierlich mit einer Eintrittsöffnung (11 a; 41 a) ausgebildet ist, durch die die Flüssigkeit in den Beutel (11; 41; 71) fließt;
einen Austrittsseitenbereich (16; 46), der kontinuierlich mit einer Austrittsöffnung (11b, 41b) ausgebildet ist, durch die die Flüssigkeit aus dem Beutel (11; 41; 71) fließt; und
einen Verbindungsbereich (17; 47), der den Eintrittsseitenbereich (15; 45) und den Austrittsseitenbereich (16; 46) miteinander verbindet,
wobei der Beutel (11; 41; 71) einen verschweißten Bereich (14b; 44b) aufweist, der durch miteinander Verschweißen der beiden Folien (14; 43, 44; 84) gebildet ist, wobei der verschweißte Bereich (14b; 44b) den Eintrittsseitenbereich (15; 45), den Austrittsseitenbereich (16; 46) und den Verbindungsbereich (17; 47) voneinander abteilt,
wobei das dreidimensionale Gewirk (12) in Form einer im Wesentlichen flachen plattenartigen Form ausgebildet ist und im Wesentlichen in dem gesamten Eintrittsseitenbereich (15; 45), Austrittsseitenbereich (16; 46) und dem Verbindungsbereich (17; 47) in dem Beutel (11; 41; 71) angeordnet ist,
wobei die von dem dreidimensionalen Gewirk (12) in dem Beutel (11; 41; 71) zurückgehaltene Flüssigkeit eine von einer kühlenden Flüssigkeit und einer wärmenden Flüssigkeit ist, und
wobei die Flüssigkeit durch den Eintrittsseitenbereich (15; 45), den Austrittsseitenbereich (16; 46) und den Verbindungsbereich (17; 47) in dem Beutel (11; 41; 71) strömt, wo das dreidimensionale Gewirk (12) angeordnet ist, **dadurch gekennzeichnet, dass** das dreidimensionale Gewirk (12) Folgendes aufweist:
eine Mehrzahl von gitterartigen Strickstücken (20), die im Wesentlichen parallel zueinander angeordnet sind; und
eine Mehrzahl von Kopplungssträngen (21) zum Koppeln der Mehrzahl von gitterartigen Strickstücken (20) miteinander,
wobei die Mehrzahl von Kopplungssträngen (21) aus elastischem chemischen Fasermaterial hergestellt ist.

2. Flüssigkeitsrückhalteeinrichtung nach Anspruch 1,
wobei der Beutel (11; 41; 71) aus dem Eintrittsseitenbereich (15; 45), dem Austrittsseitenbereich (16; 46) und dem Verbindungsbereich (17; 47) gebildet ist.

3. Flüssigkeitsrückhalteeinrichtung nach Anspruch 1 oder 2,
wobei der verschweißte Bereich (14b; 44b) im Wesentlichen T-förmig ausgebildet ist.

4. Flüssigkeitsrückhalteeinrichtung nach einem der Ansprüche 1 bis 3, wobei der Beutel (11; 41; 71) mit einer im Wesentlichen rechteckigen Form ausgebildet ist,
wobei die Eintrittsöffnung (11a; 41a) und die Austrittsöffnung (11b; 41 b) an einer in Längsrichtung einen Endseite des Beutels (11; 41; 71) mit der im Wesentlichen rechteckigen Form ausgebildet sind, und
wobei der Verbindungsbereich (17; 47) an einer in Längsrichtung anderen Endseite des Beutels (11; 41; 71) ausgebildet ist.

5. Flüssigkeitsrückhalteeinrichtung nach Anspruch 1 oder 2,
wobei die Flüssigkeitsrückhalteeinrichtung mit einer im Wesentlichen bandartigen Form ausgebildet ist und weiterhin aufweist:
ein erstes Zusammenwirkelement (49a), das an der einen Oberfläche an einer Endseite in Längsrichtung der im Wesentlichen bandartig ausgebildeten Flüssigkeitsrückhalteeinrichtung festgelegt ist; und
ein zweites Zusammenwirkelement (49b), das mit dem ersten Zusammenwirkelement (49a) zusammenwirken kann, um die eine Oberfläche an der einen Endseite in Längsrichtung der Flüssigkeitsrückhalteeinrichtung sowie eine weitere Oberfläche an einer weiteren Endseite in Längsrichtung der Flüssigkeitsrückhalteeinrichtung lösbar miteinander zu verbinden, wobei das zweite Zusammenwirkelement (49b) an der weiteren Oberfläche an der weiteren Endseite in der Längsrichtung der Flüssigkeitsrückhalteeinrichtung festgelegt ist.

6. Kühlende und wärmende Vorrichtung, aufweisend:
die Flüssigkeitsrückhalteeinrichtung nach einem der Ansprüche 1 bis 5;
eine Pumpe (7) zum Zuführen der Flüssigkeit zu der Flüssigkeitsrückhalteeinrichtung, während die Pumpe mit der Flüssigkeitsrückhalteeinrichtung verbunden ist; und
einen Wärmetauscher (8) zum Ausführen von einem von einem Kühlvorgang und einem Heizvorgang der Flüssigkeit, die der Flüssigkeitsrückhalteeinrichtung zugeführt wird.

## Revendications

1. Dispositif pour contenir un liquide, comprenant :
un sachet (11 ; 41 ; 71) possédant des propriétés de blocage d'eau et une flexibilité et façonné en forme de sachet ; et
un tricot tridimensionnel (12),
dans lequel le sachet (11 ; 41 ; 71) est façonné en forme de sachet en soudant des bords extérieurs de deux films (14 ; 43, 44 ; 84), chacun fait d'un parmi une résine et un caoutchouc, l'un à l'autre,
dans lequel le sachet (11 ; 41 ; 71) comprend :
une région côté entrée (15 ; 45) continue avec un orifice d'entrée (11a ; 41a) à travers lequel le liquide s'écoule dans le sachet (11 ; 41 ; 71) ;
une région côté sortie (16 ; 46) continue avec un orifice de sortie (11b ; 41b) à travers lequel le liquide s'écoule hors du sachet (11 ; 41 ; 71) ; et
une région de raccordement (17 ; 47) raccordant la région côté entrée (15 ; 45) et la région côté sortie (16 ; 46) l'une à l'autre,
dans lequel le sachet (11 ; 41 ; 71) comprend une partie soudée (14b ; 44b) formée en soudant les deux films (14 ; 43, 44 ; 84) l'un à l'autre, la partie soudée (14b ; 44b) séparant la région côté entrée (15 ; 45), la région côté sortie (16 ; 46), et la région de raccordement (17 ; 47) les unes des autres,
dans lequel le tricot tridimensionnel (12) est façonné en forme sensiblement similaire à une plaque plate et agencé sensiblement dans la totalité de la région côté entrée (15 ; 45), de la région côté sortie (16 ; 46), et de la région de raccordement (17 ; 47) dans le sachet (11 ; 41 ; 71),
dans lequel le liquide contenu par le tricot tridimensionnel (12) dans le sachet (11 ; 41 ; 71) comprend un parmi un liquide de refroidissement et un liquide de chauffage, et
dans lequel le liquide passe à travers la région côté entrée (15 ; 45), la région côté sortie (16 ; 46), et la région de raccordement (17 ; 47) dans le sachet (11 ; 41 ; 71) où le tricot tridimensionnel (12) est agencé,
**caractérisé en ce que** le tricot tridimensionnel (12) comprend :
une pluralité d'habillages tricotés en mailles (20) agencés sensiblement parallèles les uns aux autres ; et
une pluralité de fils simples d'accouplement (21) pour accoupler la pluralité d'habillages tricotés en mailles (20) les uns aux autres,
dans lequel la pluralité de fils simples d'accouplement (21) sont faits de fibre chimique élastique.

2. Dispositif pour contenir un liquide selon la revendication 1, dans lequel le sachet (11 ; 41 ; 71) est formé de la région côté entrée (15 ; 45), de la région côté sortie (16 ; 46), et de la région de raccordement (17 ; 47).

3. Dispositif pour contenir un liquide selon la revendication 1 ou 2, dans lequel la partie soudée (14b ; 44b) est façonnée sensiblement en forme de T.

4. Dispositif pour contenir un liquide selon une quelconque des revendications 1 à 3,
dans lequel le sachet (11 ; 41 ; 71) est façonné en forme sensiblement rectangulaire,
dans lequel l'orifice d'entrée (11a ; 41a) et l'orifice de sortie (11b ; 41b) sont formés sur un côté extrémité dans une direction longitudinale du sachet (11 ; 41 ; 71) formé en forme sensiblement rectangulaire, et
dans lequel la région de raccordement (17 ; 47) est formée sur un autre côté extrémité dans la direction longitudinale du sachet (11 ; 41 ; 71).

5. Dispositif pour contenir un liquide selon la revendication 1 ou 2, dans lequel le dispositif pour contenir un liquide est façonné en forme sensiblement similaire à une ceinture, et comprend en outre :
un premier élément d'entrée en prise (49a) fixé à une première surface sur un premier côté extrémité dans une direction longitudinale du dispositif pour contenir un liquide façonné en forme sensiblement similaire à une ceinture ; et
un second élément d'entrée en prise (49b) pouvant entrer en prise avec le premier élément d'entrée en prise (49a) pour raccorder de façon séparable la première surface sur le premier côté extrémité dans la direction longitudinale du dispositif pour contenir un liquide et une autre surface sur un autre côté extrémité dans la direction longitudinale du dispositif pour contenir un liquide l'une à l'autre, le second élément d'entrée en prise (49b) étant fixé à l'autre surface sur l'autre côté extrémité dans la direction longitudinale du dispositif pour contenir un liquide.

6. Appareil de refroidissement et de chauffage, comprenant :
le dispositif pour contenir un liquide selon une quelconque des revendications 1 à 5 ;
une pompe (7) pour fournir le liquide dans le dispositif pour contenir un liquide tout en étant raccordée au dispositif pour contenir un liquide ; et
un échangeur de chaleur (8) pour réaliser un parmi un refroidissement et un chauffage du liquide qui est fourni au dispositif pour contenir un liquide.
